# EUROPEAN PATENT APPLICATION

(11) **EP 2 924 631 A1**
(43) Date of publication of application: **30.09.2015**
(21) Application number: 15158203.8
(22) Date of filing: 09.03.2015
(51) Int. Cl.: G06Q 10/10, G05D 23/00

(54) **COMPUTER-IMPLEMENTED SYSTEM AND METHOD FOR EXTERNALLY EVALUATING THERMOSTAT ADJUSTMENT PATTERNS OF AN INDOOR CLIMATE CONTROL SYSTEM IN A BUILDING**

(30) Priority: 27.03.2014 US 201414228211
(71) Applicant: Palo Alto Research Center Incorporated, Palo Alto, California 94304 (US)
(72) Inventor: Smullin, Sylvia J., Menlo Park, CA 94025 (US)
(74) Representative: Lord, Michael

(57) **Abstract**

Energy usage data of an indoor climate control system, such as an HVAC system, for a building and ambient temperature data are obtained for a time period of interest with a time resolution that reflects the physically relevant time scales. The data are formed into time series. A correlation between the system's usage and ambient temperature is established, where a strong (or high) correlation is interpreted as an indication that the thermostat set point infrequently gets changed, if at all, whilst a weak (or low) correlation is interpreted as an indication that the thermostat set point is changed regularly. In addition, a correlation between the system's usage and the building's occupancy can be established, which can help corroborate the assessment of the appropriateness or efficiency of thermostat set point changing patterns.

## Description

### Field

This application relates in general to indoor climate control within a building, and in particular, to a computer-implemented system and method for externally evaluating thermostat adjustment patterns of an indoor climate control system in a building.

### Background

Systems to control indoor climate are commonly found in residential, commercial, retail, and industrial buildings. Whether in the form of a dedicated cooling- or heating-only system, or a combined heating, ventilation and air conditioning (RV AC) system, indoor climate control systems serve two main purposes. First, these systems help maintain thermal comfort for occupants of a building by heating, cooling, or ventilating air within a structure relative to ambient temperatures and conditions. Second, these systems help to improve air quality through filtration of airborne particulates, provide isolation from outdoor environments, and remove humidity from the air.

The operation of an indoor climate control system is normally controlled via a thermostat or similar indoor controller that measures indoor temperature and regulates the On- and Off-cycling of the system components to maintain the indoor temperature around the thermostat set point. Typically, each operating cycle includes a time during which the system is running continuously as necessary to bring the building's interior temperature into a temperature range defined about a desired indoor temperature followed by time during which the system is at idle or on standby.

Indoor climate control system usage contributes significantly to the energy consumption of a building, especially when operated in an inefficient manner. From the perspective of an energy consumer, running an indoor climate control system unnecessarily, such as when a building is unoccupied, or without concern to thermal comfort need, for instance, at the same thermostat set point regardless of whether people are present or active, can have a negative effect on the overall cost of energy used. Nevertheless, the average residential consumer may lack a sufficient incentive to make changes purely to save on a monthly energy bill, whether by upgrading the structural aspects of a building, modifying their indoor climate control system usage behaviors, or modifying other behaviors, such as uses of other appliances that impact the usage of the indoor climate control. On the other hand, while reducing energy consumption remains discretionary for most consumers, power utilities may be under a compulsory mandate to urge consumers to reduce the amount of energy used in an effort to balance an ever-increasing demand for more energy, or lowering consumer energy consumption may simply make good fiscal sense, for example, to help a utility save or defer capital expenditures for building new power generation plants, running transmission lines, and upgrading infrastructure.

Frequently, power utilities urge consumers to reduce energy consumption through educational and compensatory outreach programs, which can include incentives, rewards, advice, outreach, education, and other forms of offerings to the consumers. For example, some power utilities offer rebates to incent consumers to make structural changes, such as switching to compact fluorescent lights or increasing thermal insulation. Problematically, such rebates may not always be awarded to those consumers who would benefit most and may not always incent structural or behavioral changes that will have the most impact on energy savings for each consumer, such as when rebates are offered to all consumers on a first-come, first-served basis.

Consumers who are "energy outliers," that is, consumers who use considerably more energy than neighboring or comparable consumers, are better targets for energy consumption reduction incentives, although they may be unaware of their outlier status or of what changes are needed to help reduce their energy consumption. House energy audits can help consumers to prioritize the changes necessary to become more energy efficient, but energy audits are often costly and they do not account for how human behavior and the operation of the indoor climate control system can vary over time. Moreover, monitoring consumers for reductions in energy consumption after changes have been made is difficult. Energy usage must be measured over long time periods, and weather, behavioral, and structural factors bearing on energy usage must be deconvoluted from the energy usage measurements to correlate energy consumption reduction incentives to realized energy savings. Without visibility into or understanding of all these compounding factors that determine energy usage, the savings accomplished by rebate programs may be calculated as deemed savings, rather than directly measured or assessed.

As used herein, an energy outlier is a consumer who uses more energy for indoor climate control than other comparable consumers, where the comparison may include a normalization for size of house, cooling degree days or heating degree days, the number of occupants in a house, or other information. A consumer that uses more energy for indoor climate control in a house than most others may use more energy for one or more of the following reasons: an extraordinary thermostat set point, malfunctioning or poor placement of the thermostat, malfunctioning of the climate control system due to poor maintenance or other failure, incorrect sizing of the climate control system for the building, poor thermal insulation, large effective leak area between the building and the outdoors, high internal loads due to occupants and appliance usage, or other reasons. Understanding of the relative thermal performance of houses and usage of the indoor climate control system can be used for better targeting and assessing incentive programs related to reduction of energy consumption for indoor climate control. The recipients of incentive programs will generally be the person or entity most directly connected with being able to actually use the form of incentive offered, and not the building or house proper. For example, a homeowner living in his house who is also a customer of a power utility is usually responsible for setting the thermostat in his house. If the house has a very low thermostat set point during a summer cooling season, incenting that customer to change the thermostat set point may be cheaper and more effective than incenting that same customer to blow new insulation into the walls, which he, as a homeowner, could do. In contrast, only the owner of an apartment building would likely be able to make structural changes to the building, rather than any tenants or his property management company, which generally only maintains and repairs, and does not ordinarily replace or upgrade, building indoor climate control systems and structure, like indoor climate control systems or wall insulation, or other capital improvements.

Indoor temperature, as reflected by a thermostat set point, is a key determinant in the amount of energy used for indoor climate control, particularly during the cooling and heating seasons. The need to supply indoor climate control for maintaining thermal comfort is primarily dictated by the presence, or absence, of occupants. In standalone houses, the thermal load on an indoor climate control system can be expected to be mainly due to ambient temperature and conditions and less dependent on occupancy. In commercial, retail, and industrial spaces, the thermal load may be determined more strongly by occupancy or appliance and machinery usage, although appliance and machinery usage may contribute a relatively constant and predictable thermal load.

In both classes of buildings, indoor climate control systems may still be run during periods of time when heating or cooling is not needed. Whether on purpose or due to inattention, inaction or naivety, occupants who neglect to adjust their thermostats to suspend system operation when occupancy changes, for instance, when a house is empty, or other heating or cooling needs change, needlessly waste energy. Currently, power utilities or third parties only know whether thermostat set points have been changed if occupants report the settings or use a "smart" thermostat that collects and reports such information. As a result, there are only limited circumstances under which buildings with occupants having inefficient thermostat "habits" can be identified from outside and addressed.

Therefore, there is a need for an approach to determining whether an indoor climate control system is being used in an efficient manner that reflects regular adjustment of thermostat set point in response to actual thermal comfort need.

### Summary

Energy usage data of an indoor climate control system, such as an HVAC system, for a building and ambient temperature data are obtained for a time period of interest with a time resolution that reflects the physically relevant time scales. The data are formed into time series. A correlation between the system's usage and ambient temperature is established, where a strong (or high) correlation is interpreted as an indication that the thermostat set point infrequently gets changed, if at all, whilst a weak (or low) correlation is interpreted as an indication that the thermostat set point is changed regularly. In addition, a correlation between the system's usage and the building's occupancy can be established, which can help corroborate the assessment of the appropriateness or efficiency of thermostat set point changing patterns.

One embodiment provides a computer-implemented system and method for externally evaluating thermostat adjustment patterns of an indoor climate control system in a building based on temperature. A usage time series that reflects indoor climate control system usage in a building over a plurality of operating cycles is obtained. Each operating cycle includes a "go-to-idle" state transition during which the indoor climate control system transitions from a running state to an at idle state and a "go-to-run" state transition during which the indoor climate control system transitions from an at idle state to a running state. The indoor climate control system runs for a period of running time between each "go-to-run" state transition and the next "go-to-idle" state transition, the indoor climate control system remaining at idle for a period of idle time between each "go-to-idle" state transition and the next "go-to-run" state transition. The running time is the time necessary to bring the building's interior temperature into a temperature range defined about a desired indoor temperature for the building. A temperature time series for the temperature ambient to the building over the same plurality of the operating cycles is obtained. A temperature correlation between the usage time series and the temperature time series is found, wherein a low temperature correlation is interpreted as changing of the thermostat set point during the operating cycles.

A further embodiment provides a computer-implemented system and method for externally evaluating thermostat adjustment patterns of an indoor climate control system in a building based on temperature and occupancy. A usage time series that reflects indoor climate control system usage in a building over a plurality of operating cycles is obtained. Each operating cycle includes a "go-to-idle" state transition during which the indoor climate control system transitions from a running state to an at idle state and a "go-to-run" state transition during which the indoor climate control system transitions from an at idle state to a running state. The indoor climate control system runs for a period of running time between each "go-to-run" state transition and the next "go-to-idle" state transition, the indoor climate control system remaining at idle for a period of idle time between each "go-to-idle" state transition and the next "go-to-run" state transition. The running time is the time necessary to bring the building's interior temperature into a temperature range defined about a desired indoor temperature for the building. A temperature time series for the temperature ambient to the building over the same plurality of the operating cycles is obtained. An occupancy time series for occupancy of the building over the same plurality of the operating cycles is obtained. A temperature correlation between the usage time series and the temperature time series is found, wherein a low temperature correlation is interpreted as changing of the thermostat set point during the operating cycles. An occupancy correlation between the usage time series and the occupancy time series is found, wherein a high occupancy correlation coupled with the low temperature correlation is interpreted as the thermostat set point changing with changing of the occupancy of the building.

The foregoing approach fundamentally gets at *why* one building might be using more energy for indoor climate control system usage than others by narrowing the set of possible reasons and showing which of the buildings might have thermostat set point usage patterns that are less efficient than the patterns found in other buildings. The comparison between houses of their patterns of adjusting their thermostat set points could be used in conjunction with other comparisons of thermal performance of house or occupant behavior to determine which houses would most likely benefit from what incentives being provided to their owner, occupant, or responsible party. If a power utility wants to offer rebates or incentives related to thermostats to the owner, occupant, or responsible party, the utility might, for example, choose to make the offer first to those owners, occupants, or responsible parties of houses who, by neglecting to adjust their thermostats to suspend system operation when occupancy changes or other heating or cooling needs change, needlessly waste energy.

In addition to being used to select which owner, occupant, or responsible party of a house to receive a particular incentive, the foregoing approach could further be used as part of a study of a group of houses, including identifying the top reasons why one house uses more energy than others and choosing the best incentives for the owner, occupant, or responsible party of that house. If a power utility wants to offer an incentive to the owner, occupant, or responsible party of one house to make some behavioral or structural change, the foregoing approach could be used to choose what kind of behavioral or structural change to incent for that one house by way of their owner, occupant, or responsible party.

Finally, the foregoing approach could be used to monitor the impact of any energy efficiency program by making the same comparison before and after any incentive program to the owner, occupant, or responsible party of a building has been implemented.

Still other embodiments of the present invention will become readily apparent to those skilled in the art from the following detailed description, wherein is described embodiments of the invention by way of illustrating the best mode contemplated for carrying out the invention. As will be realized, the invention is capable of other and different embodiments and its several details are capable of modifications in various obvious respects, all without departing from the spirit and the scope of the present invention. Accordingly, the drawings and detailed description are to be regarded as illustrative in nature and not as restrictive.

### Brief Description of the Drawings

FIGURE 1 is a functional block diagram showing factors affecting the operation of an indoor climate control system in a house.
FIGURES 2 and 3 are graphs respectively showing, by way of examples, cooling power and heating power as functions of indoor temperature.
FIGURE 4 is a flow diagram showing a computer-implemented method for externally evaluating thermostat adjustment patterns of an indoor climate control system in a building in accordance with one embodiment.
FIGURE 5 is a flow diagram showing a routine for correlating indoor climate control system usage to ambient temperature for use in the method of FIGURE 4.
FIGURE 6 is a flow diagram showing a routine for correlating indoor climate control system usage to occupancy for use in the method of FIGURE 4.
FIGURE 7 is a block diagram showing a computer-implemented system for externally evaluating thermostat adjustment patterns of an indoor climate control system in a building in accordance with one embodiment.

### Detailed Description

An "energy outlier" is a consumer of a power utility who uses considerably more energy for indoor climate control system operation than neighboring or comparable consumers under similar ambient temperatures and conditions. There are many reasons why a consumer may be an energy outlier for indoor climate control system usage. For air conditioning, the reasons may be due to an overly-low thermostat set point; an overly-high internal thermal load due to high occupancy, lots of cooking activity, or large numbers of appliances turned on; using air conditioning in a wasteful manner, such as leaving the system running when nobody is around, a leaky house due to poor insulation; or some other reason.

Understanding why energy outliers are outliers can help a power utility in deciding which incentives or education to offer to what customers. Power utilities in the residential energy market lack sufficient tools for designing rebate or other incentive offerings and verifying the impact of such rebates or incentives on energy consumption. The discussion that follows provides a power utility with a set of tools that can be used to identify and narrow the set of reasons underlying why a consumer is an energy outlier, so as to help choose energy consumption reduction incentives to offer to encourage structural or behavioral changes. For example, does the house of an energy outlier have poor insulation? Lots of unshaded windows? An extraordinary thermostat set point? An improperly-sized indoor climate control system? System usage that never varies, despite changes in occupancy and, therefore, thermal comfort needs? A detailed energy audit can answer some of these questions. Here, the goal is to deduce what is possible with a limited set of information about the house, specifically, energy usage data that indicates when the indoor climate control system is running or at idle (or on standby), that is, cycling On and Off, and weather information for the same time period as the energy usage data.

These same tools can be used to choose those customers of a power utility to whom to offer a particular incentive by narrowing the set of customers that are most likely to receive maximal energy consumption reduction benefit from the incented change. Much of the discussion is presented in the context of energy use for cooling with air conditioning; however, except as specifically related to humidity, the discussion can equally be applied *mutatis mutandis* to energy use for heating or other forms of indoor climate control that involve the use of a thermostat or other temperature-based control system.

Energy usage is directly related to the running time of an indoor climate control system. In turn, running time and efficient operation are related through thermostat set point, system sizing, thermostat set point adjustment, and other factors. Knowledge of an indoor temperature is crucial for understanding a building's energy use for indoor climate control; such understanding is a key determinant of the amount of heat that needs to be added by a heater or removed by an air conditioner. An effective thermostat set point, as a surrogate of indoor temperature, can be externally inferred based on system usage and ambient temperature data, such as described in commonly-assigned U.S. Patent application, entitled "Computer-Implemented System and Method for Externally Inferring an Effective Indoor Temperature in a Building," Serial No. 14/228207, filed March 27, 2014, Docket No. 20131644US01, pending, the disclosure of which is incorporated by reference. In addition, an indoor climate control system may run less efficiently upon being turned on, and thus the overall efficiency will be greater when the running times are longer and the system cycles fewer times. The apparent sizing of the system can also he extrinsically inferred, such as described in commonly-assigned U.S. Patent application, entitled "Computer-Implemented System and Method for Externally Evaluating Sizing of an Indoor Climate Control System in a Building," Serial No. 14/228,209, filed March 27, 2014, Docket No. 20131645US01, pending, the disclosure of which is incorporated by reference.

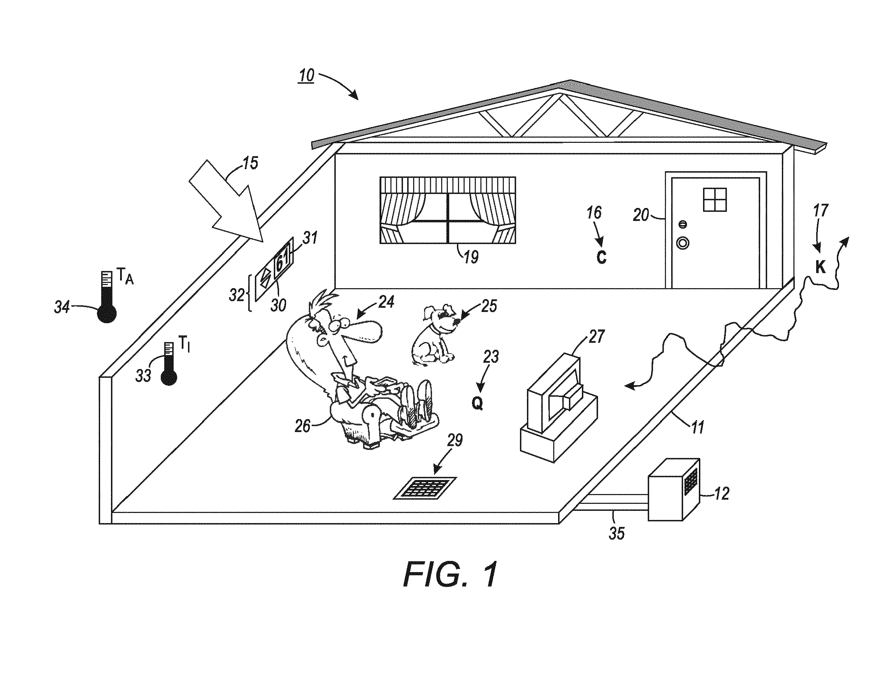

The efficiency contributions of an appropriate thermostat set point, proper system sizing, and other factors can be negated through inefficient indoor climate control system usage; regular adjustment of thermostat set point in response to actual thermal comfort need can significantly contribute to efficient indoor climate control system operation. Indoor climate control system usage is influenced by several factors. FIGURE 1 is a functional block diagram showing factors 10 affecting the operation of an indoor climate control system 12 in a house 11. Although described herein with specific reference to a house 11, the same or similar factors affect the operation of other types of buildings, including commercial, industrial, and so forth, except as otherwise noted.

Effective indoor temperature 33, as reflected by a thermostat set point (Tₒ) 31, is one factor affecting system running time and, therefore, the amount of energy used for indoor climate control, particularly during the cooling and heating seasons, which can also provide an explanation as to why some houses may use more energy for indoor climate control than their neighbors, peers, or comparable customers. Herein, the terms neighbors, peers, and comparable customers can be used interchangeably.

For purposes of discussion, operation of the indoor climate control system 12 is assumed to be manually controllable of the house 11 via the thermostat 30, or similar indoor controller that measures indoor temperature, which provides a temperature controlling component to the indoor climate control system 12, and regulates the On- and Off-cycling of the system components between running and at idle (or on standby) to maintain the indoor temperature around the thermostat set point 31. The thermostat 30 will be assumed to control only one climate-controlled zone and one climate-control system, in comparison to buildings that have multiple zones or multiple climate-control systems, although the same inferences described herein would, by extension, similarly apply to such configurations. The thermostat set point 31 can be manually adjusted from within the house 11 by the occupants using controls 32 provided with the thermostat 30, automatically adjusted by a thermostat that learns occupant behavior, can be set to change on a timer, or could respond to other conditions, such as indoor humidity or occupancy. As well, the thermostat set point 31 may also be adjustable from outside of the house 11 by other people using controls that are remotely interfaceable to the indoor climate control system 12, such as performed centrally by a facilities manager in charge of campus-wide climate control, or by a smart thermostat that includes wireless capabilities that allow remote control via a smartphone or other device. Other thermostat set point 31 modes of adjustment or operation are possible.

Data showing usage of an indoor climate control system must be available for the house 11, specifically the times at which the system is running and at idle or on standby. Data showing the actual power used by an indoor climate control system, though, is not essential and may be of secondary consideration.

Ambient temperature information for the same time period as covered by the indoor climate control system usage data must be available for a geographic area that includes or represents the location of the house 11. Natural variations in weather provide continually-varying experiments on each building. Ambient temperature may be the outdoor dry bulb temperature, the sol-air temperature, or a temperature measured on the building envelope.

Information about indoor climate control system usage must be separated from other energy usage in a house. For the inference of changing thermostat set points described herein, only the time that an air conditioner is running and has gone to idle or standby, rather than the total energy draw, needs to be considered. For an air conditioner, which is generally expected to have cooling power and coefficient of performance (COP) that vary with outdoor temperature, this usage time series may have different trends than the power or total energy draw that is commonly used in other analyses; for an electric resistive heater, the amount of energy drawn is expected to be close to directly proportional to the time that the heater is turned on. System usage data can be derived from various sources. For instance, many power utilities are currently installing "smart" power meters that monitor energy usage at 1-hour or 15-minute time resolutions. Monitoring at still higher time resolutions, such as at one-minute intervals, can be one way of indirectly determining when an air conditioner runs and goes to idle or standby in a house 11 over the course of a cooling season, particularly as cycles of air conditioners in houses are often on the order of 5-20 minutes in duration, provided a disaggregation algorithm is applied to the meter data to separate-out air conditioning usage. Smart meters and disaggregation algorithms can provide new data streams to power utilities and other users, enabling tools, such as described herein. Still other sources of indoor climate control system usage data are possible, including a meter that monitors the electrical current to an air conditioning circuit alone, a sensor that monitors air flow in the duct work, a sensor that measures the vibration of the air conditioning when running, and usage reported by smart thermostats. The total amount of electricity, gas, or other fuel used over a period of time for an indoor climate control system can also be used as a proxy for the fraction of time that a system is running, even if usage is not monitored with a time resolution that is high enough to distinguish the exact minute during which the climate control system cycles runs or goes to idle or standby. The fidelity of this approximation depends on how much the efficiency of the indoor climate control system varies with ambient temperature and the time resolution at which fuel use is monitored.

Throughout the day, in addition to possible manual adjustment of the thermostat 30 by occupants of the house 11, or others, several factors 10 can affect the operation of an indoor climate control system 12. These factors include the heat capacity C (16) of the house 11, the thermal conductance K (17) of the house 11 between the interior and the exterior, an interior heat load *Qᵢₙ* (23) generated within the house 11, and a non-temperature-driven heat load *Qₒᵤₜ* from outside the house 15. Sources of heat load *Qᵢₙ* include the heat generated by people 24, pets 25, furnishings 26, and operating appliances 27 located in the house 11, which can cause the heat load *Qᵢₙ* to continually change. External heat loads that are not related to the temperature difference between indoors and outdoors include the latent load due to infiltration. Loads due to radiation from the sun and radiative coupling to the environment may be included in either *Qₒᵤₜ* or may be coupled into the term proportional to the temperature difference. Radiation may also be incorporated into the model, using knowledge or assumptions about the building type, structure, and location, by using the sol-air temperature as the ambient temperature, instead of the outdoor dry bulb temperature, such as described in the 2013 ASHRAE Handbook, Fundamentals, SI Ed. (2013).

In addition, in the case of air conditioning, ambient temperature that is higher than the indoor temperature creates a load via thermal conduction from outside to inside. In the case of heating, ambient temperatures that is lower than the desired indoor temperature is of importance. The ambient temperature also creates a load via infiltration when leaks in the house, ventilation systems, open doors or windows, or chimneys allow outdoor air to come inside a house. Radiation between the surroundings, including the ground, the sky, and the entirety of the outdoor environment, also create a means for heat exchange between the outdoors and the building envelope, with further heat exchange between the building envelope and the indoor air determining the load on the interior of the house.

In the simplified model described herein, the effective thermal conductance *K* can encompass all temperature-dependent sensible heat transfer between the indoor and outdoor environment through the building envelope due to conduction, infiltration, convection, and radiation, which, in some cases, may be appropriately linearized. The thermal conductance *K* determines the temperature-dependent heat transfer between the interior of the house 11 at temperature *T* (33) and the ambient temperature outside of the house *T_{A}* (34). In this model, there is no spatial variation of *Tor T_{A},* and no explicit convective or radiative heat transfer. More generally, *K* can be considered to be an overall heat loss coefficient that reflects properties of the building envelope.

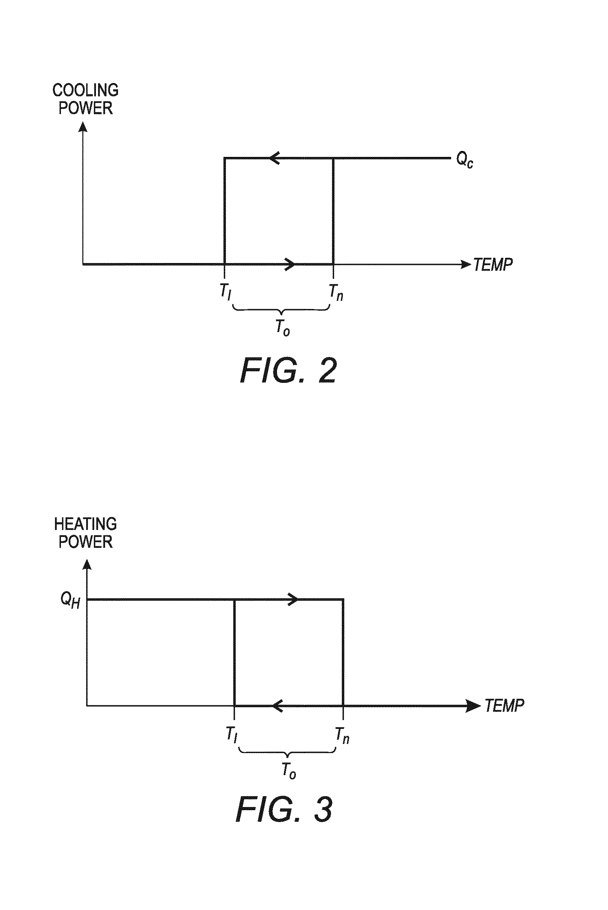

In the model, the thermostat 30 of the house 11 is set to maintain the interior temperature 33 at *T* = *T₀,* where *T₀* is the set point chosen for the thermostat 30. The thermostat 30 operates using a "deadband." FIGURES 2 and 3 are graphs respectively showing, by way of examples, cooling power (for air conditioning) and heating power (for heating) as functions of indoor temperature. For simplicity, any variations in system cooling power or heating power with temperature are ignored in the graphs, which are intended to convey a simple version of a thermostat control scheme. To avoid constant cycling between running and at idle (or on standby) modes, most thermostats operate using a "deadband" that begins at a temperature *Tₗ* slightly below and ends at a temperature *Tₕ* slightly above the thermostat set point *To.* An indoor climate control system will start running when the indoor temperature falls below the deadband (for heating) or rises above the deadband (for cooling). Obversely, the system will go to idle or standby when the indoor temperature rises above the deadband (for heating) or falls below the deadband (for cooling). Though indoor climate control systems may have control schemes that are more complicated, this deadband model captures the most important features of thermostat operation.

Referring back to FIGURE 1, more precisely, an air conditioning system will start running when *T* ≥ *Tₕ = T₀* + *dTₕ* and will go to idle or standby when the AC when *T* ≤ *Tₗ = T₀ - dTi,* where *dTₕ* and *dTₗ are* upper and lower offsets that define the deadband of the thermostat 30, and *To* is the thermostat set point. In an indoor climate control system, this deadband may be set in the thermostat alone, in some other component of the system, or by a combination of settings in the thermostat and one or more other components of the system. This behavior may also be called *hysteresis* in the system, and, in this context, the deadband may also be called a *hysteresis band* or the tolerance of a thermostat. The mechanical device that accomplishes this effect may be, for example, an anticipator in an analog thermostat or a control built into a microprocessor in a digital thermostat.

Absent manual system cutoff at the thermostat 30 by the occupant of the house 10 or others, the duration of the running time of an air conditioner depends on its cooling power, the sum of the loads, the thermal properties of the building, and the size of the deadband. The sizing of the indoor climate control system refers to the comparison of the cooling power (for an air conditioner) or the heating power (for a heater) in relationship to the loads experienced. The thermostat 30 is generally located in a place that is likely not the coldest part of the system. In this model, the details of the thermal network of the air conditioning, ductwork, and forced convection are factored into the cooling power *Q_{c}*, where the cooling power *Q_{c}* represents the cooling power that is experienced by the thermostat 30. Here, only sensible power is considered, as most thermostats respond only to temperature.

This model is highly simplified in considering the house as one lumped thermal mass. In a real house that has a thermal environment much more complex than what is described herein, the conduction load is expected to be the dominant heat load with the most variance. This expectation is reflected in analyses of indoor climate control system usage based on heating degree days and cooling degree days, such as described in the ASHRAE Handbook, cited *supra,* and the expected substantially linear relationship between duty cycle and ambient temperature, such as described in commonly-assigned U.S. Patent application, entitled "Computer-Implemented System and Method for Externally Inferring an 30 Effective Indoor Temperature in a Building," Serial No. 14/228,207, cited *supra.*

For a fixed thermostat set point, if the load proportional to the temperature difference between indoors and ambient is the largest thermal load on the house, air conditioning usage will show a strong correlation to ambient temperature variations over time. If other loads are large and varying, or if the thermostat set point is changed, this correlation will be weaker. In the context of house, when the correlation between ambient temperature and air conditioning usage is weak, an indication that the thermostat set point is changed at certain points in time is considered the most likely reason for the weak correlation. Averaging over repeating periods of time can accentuate the lack of correlation between usage and ambient temperature when discrete changes in thermostat set point occur, for example, at approximately the same time every day.

While for the case of air conditioning, usage is expected to increase with temperature and the correlation between usage and temperature is expected to be positive, for the case of heating, usage is expected to increase as temperature decreases, which would result in a negative correlation between temperature and usage. In the case of air conditioning, a strong temperature correlation is positive and a weak temperature correlation is one that is a small positive correlation or a negative correlation. In the case of heating, a strong temperature correlation is a negative correlation and a weak temperature correlation is a positive correlation or a negative correlation that is small in magnitude.

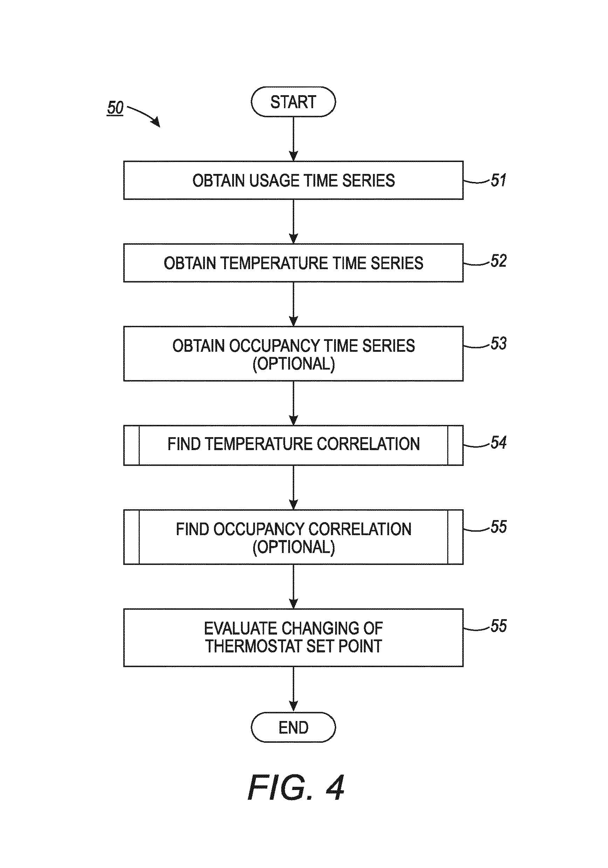

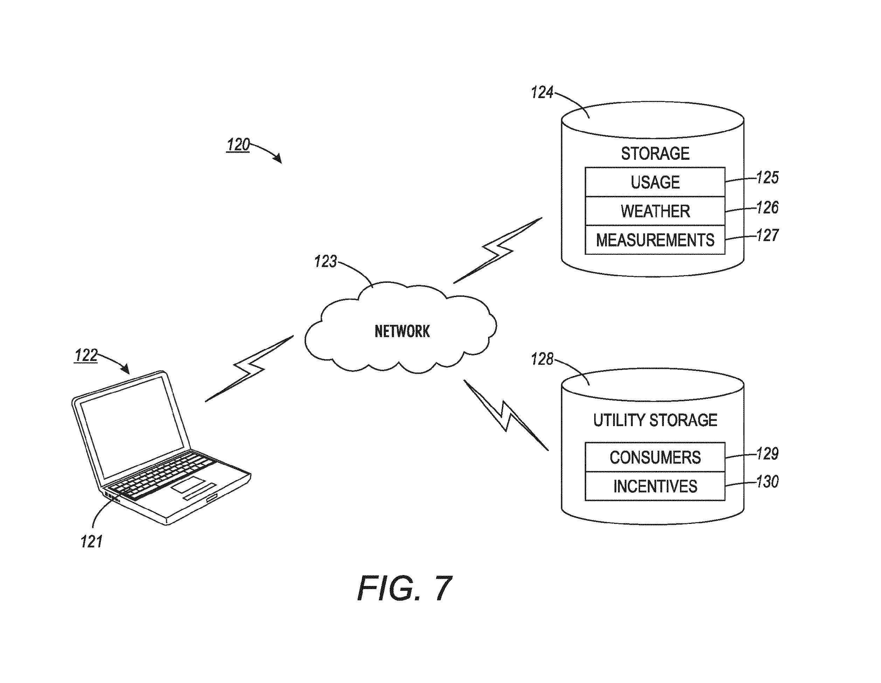

Subject to the foregoing considerations, indoor climate control system patterns of usage indicative of manual adjustment of the thermostat of a building, as occurring in a manner possibly unrelated to ambient temperature, can be determined. In a further embodiment, the usage patterns can be evaluated in the context of changes in occupancy. FIGURE 4 is a flow diagram showing a computer-implemented method 50 for externally evaluating thermostat adjustment patterns of an indoor climate control system in a building in accordance with one embodiment. The method is performed as a series of process or method steps performed by, for instance, a general purpose programmed computer, such as further described *infra* with reference to FIGURE 7.

Only a limited set of information about the building is needed to externally identify indoor climate control system usage patterns. First, a time series of indoor climate control system usage data, which reflects usage of the system in the building over several operating cycles, is obtained (Step 51). The data in the usage time series can be expressed as binary indications of whether the indoor climate control system is running or at idle at any given time. Second, a time series of weather information, specifically, ambient temperature *T_{A},* for the same operating cycles as the usage time series, is obtained (Step 52). Preferably, the time series include enough data for study over a long enough period of time to include the natural variations in ambient temperature and indoor climate control system running conditions as needed to make a sound analysis. In a further embodiment, a times series of occupancy data that shows the number of people present in the building throughout various times of the day is obtained (Step 53).

The occupancy data could reflect all people in the building, or only the occupants of the building who are awake or exhibiting a given level of activity, for instance, actively moving about the building, as opposed to sitting in one place.

The usage time series data can be processed in operating cycles with each operating cycle characterized by a duty cycle. For each operating cycle, a duty cycle is the ratio of the time that the system is running to the total time of the operating cycle. An operating cycle is defined as a window of time during which the indoor climate control system undergoes two discrete state transitions, a "go-to-idle" state transition during which the system transitions from a running state to an at idle or on standby state, and a "go-to-run" state transition during which the indoor climate control system transitions from an at idle or on standby state to a running state. The indoor climate control system runs for a period of time between each "go-to-run" state transition and the next "go-to-idle" state transition, and the indoor climate control system remains at idle or on standby for a period of idle time between each "go-to-idle" state transition and the next "go-to-run" state transition. The ambient temperature at which an air conditioner first starts running suffers from a delay between the time that ambient temperature changes and the time that the load is experienced by the air conditioner. The system runs for the time necessary to bring the building's interior temperature into a temperature range defined about or around a desired indoor temperature for the building. The window of time can be shifted forward or backward, such that an operating cycle begins while the system is running, then transitions to at idle or on standby, and subsequently starts running again, or while the system is at idle or on standby, transitions to running, and subsequently goes to idle or on standby again, so long as the shifting of the window of time is consistent for all operating cycles.

Either or both of the raw usage time series and the temperature time series can be filtered before the correlation is assessed. Optionally, one or both of the time series can be divided into multiple spans of time and, if desired, a different filter can be selected for each time span. One or more filters can be applied to one or both of the time series, such as an exponential filter, a moving average filter, a weighting moving average filter, a low pass filter, a band pass filter, and a noise smoothing filter. As well, a time delay filter could be chosen to introduce a lag into the temperature time series, as compared to the usage time series. The exponential filter can account for the exponential decay of temperature with time, for example, in this simple thermal model of the house. In one embodiment, the usage time series and the temperature time series are both filtered with an exponential filter to reflect the physical time scale of the house. The time constant of the filter is adjusted based upon information about the type, size, or construction of the house. The time constant may also be adjusted to improve the statistical analysis. The time constant may further be chosen as a value that is considered typical for houses of a certain type or may be chosen to be either much smaller or much larger than is considered to be typical, to reduce bias when the exact time constant is unknown. Other filters or combinations of filters are possible. Use of such filters is enabled by having data at a time resolution appropriate to the physical time scales of the system.

The usage data and the temperature time series may also each be scaled by some factor. For example, as the cooling power of an air conditioner is expected to change with outdoor temperature, the usage time series data could be scaled by a function of the ambient temperature at an appropriate time to account for this physical effect.

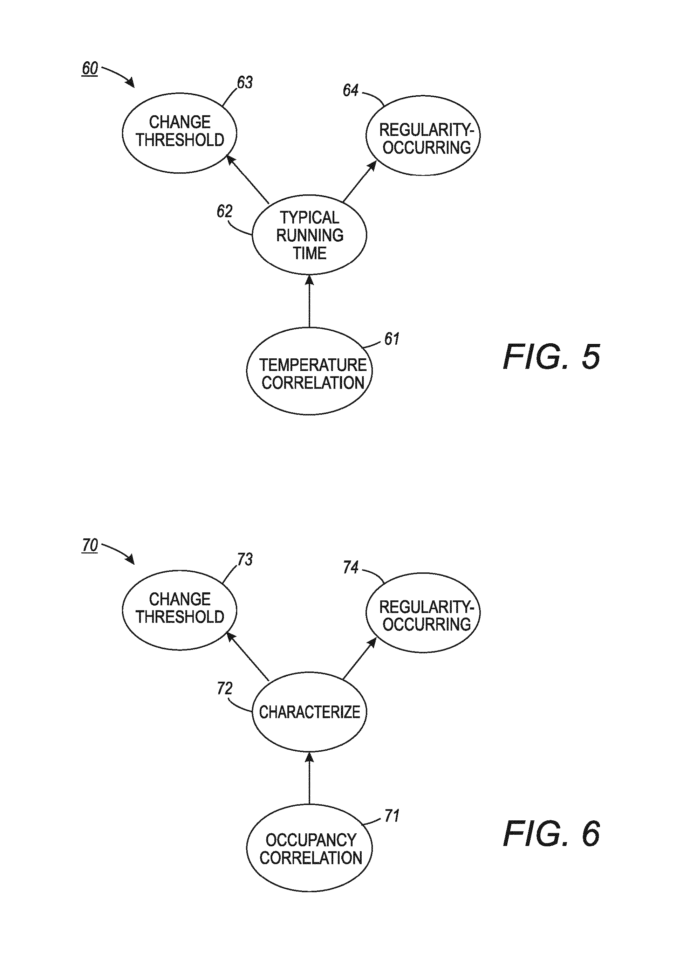

After any selection, filtering, or scaling, the usage time series data and temperature time series data are evaluated to find a correlation between the usage of the indoor climate control system and the ambient temperature (Step 55), as further described *infra* with reference to FIGURE 5. A high correlation between system usage and ambient temperature indicates that the thermostat set point was not significantly changed over the course of the time period; whereas, a low correlation indicates that the thermostat set point was adjusted regularly. Assessing any adjustments of the thermostat set point from cycles over a full day, or even better, over many days or weeks, leads to a more accurate assessment of the adjustment patterns over that time period. As well, the usage time series data or the temperature time series data can be interpolated, rounded, or summed to a different time base, such as interpolating hourly temperature data to match the higher time resolution usage data or adjusting minute-resolution usage data by rounding or summing to five-minute resolution. Further, data can be included (or excluded) by selecting operating cycles in the usage time series for only certain hours days, weeks, months, times of the year, or time periods, along with the corresponding temperatures in the temperature time series. In this way, weekday patterns can be separated from weekend patterns, and vacations or other extraordinary days may be excluded from the analysis.

As discussed *supra,* in a house, air conditioner usage and ambient temperature are expected to be strongly correlated due to the dominance of the conduction load. If left unadjusted, an air conditioner will operate to maintain the indoor temperature around the thermostat set point in a predictable fashion. However, where the usage varies with respect to ambient temperature, other factors may be affecting the system's operation, including manual adjustment of the thermostat set point. FIGURE 5 is a flow diagram showing a routine for correlating indoor climate control system usage to ambient temperature 60 for use in the method 50 of FIGURE 4. The correlation between usage and temperature can be established through various statistical analyses (step 61), including characterizing the correlation as a Pearson product-moment correlation coefficient, a rank correlation coefficient, a multi-moment correlation coefficient, or a Brownian or distance correlation coefficient. The usage time series implicitly reflects a time delay incurred relative to the ambient temperature time series due to the temporal sequence of ambient temperature change to eventual indoor climate control system reaction. A reverse delay, where the ambient temperature time series reflects a time delay, instead of the usage time series, is also possible. Consequently, the correlation could represent a cross-correlation or a coefficient of correlation between a time series that includes a time delay relative to the other time series. Finally, the correlation could be expressed as a coefficient of correlation between a finite difference or a derivative taken of the data in one of the time series and the finite difference or a derivative taken of the data in the other time series. Still other types of correlation are possible.

In addition, the correlation can be established by further characterizing the data in the usage time series and the temperature time series (step 62). A time unit that is larger than the time unit used in the usage time series is selected; and the usage time series is divided into increments of the selected time unit. The usage data falling into each increment of the selected time unit are formed into groups from which characteristic usages that are representative of each usage data group are found. The characteristic usages and the characteristic temperatures can be defined, for example, as a mean, a median, a mode, and a percentile. Similarly, the temperature data falling into each increment of the selected time unit are formed into groups from which characteristic temperatures that are representative of each of the temperature data group are found. The correlation is then defined as a function of the characteristic usages of each usage data group and the characteristic temperatures of each temperature data group. The function can be, for instance, a statistical correlation of characteristic usage and characteristic temperature. In this way, the correlation may be assessed, for example, between median usage and mean temperature over each half hour of the time period of the time-series.

In a further embodiment, the characteristic usages and characteristic temperatures can be screened using thresholds (step 63) that define a minimum amount or degree of change that must be respectively observed in consecutive characteristic usages and characteristic temperatures. The characteristic usages and characteristic temperatures that exceed their thresholds of change are formed into time series from which the correlation is drawn. Sharp changes in the ambient temperature are expected to be rare, and sharp changes in the usage are most likely indicators of changes in the thermostat set point, for example, when the air conditioning thermostat set point is lowered when people return to a house after being away during the workday.

In a still further embodiment, the characteristic usages and characteristic temperatures can be evaluated for regularly-occurring changes (step 64). Another time unit that is bigger than and an even multiple of the originally selected time unit is used. For example, if the originally selected time unit is a half hour time period, the bigger time unit could be a 24-hour period, that is, one day, and each half hour time period within a day is then treated as a slot to which the characteristic usage or characteristic temperature is assigned. In an even further embodiment, the originally selected time unit can be adjusted to account for a shift of daylight savings time, daylight standard time, or due to seasonal daylight periods. For example, the half-hour time slots in each day may be adjusted to account for the changing time of sunrise and sunset, since insolation is a large determinant of ambient temperature. The bigger time units are arranged into an ordered sequence that include the original time series; for instance, the three months of the summer are composed of a set of 92 bigger time units that are each a one-day period. A repeating time series is formed by dividing the usage time series by the bigger time unit and further dividing each bigger time unit into the originally-selected time unit; in this example, the usage time series is broken into one-day periods, and each one-day period is a repeating series of half-hour periods. The characteristic usages and the characteristic temperatures of the originally-selected time units that occur in the same order of each bigger time unit are collected from the repeating time series and aggregated. For example, if each one-day period represents 48 half-hour periods, a collection of all of the data in the first time unit in each one-day period corresponds to the first half hour of each day. For all the days in the time-series being analyzed, temperature data for that first half hour of each day is aggregated in the first of the 48 groups and usage data from that first half hour of each day is similarly aggregated. A characteristic temperature is drawn from each temperature group, and a characteristic usage is drawn from each usage group. The correlation is then defined as a function of the 48 aggregated characteristic usages and the 48 aggregated characteristic temperatures. The function can be, for instance, a statistical correlation of characteristic usage and characteristic temperature. This binning technique, which is similar to what is described in Reed, John H., "Physical and Human Behavioral Determinants of Central Air-Conditioner Duty Cycles," 1991 Energy Prog. Eval. Conf. (1991), the disclosure of which is incorporated by reference, can highlight patterns, such as a thermostat set point that is changed at the same time on every weekday and the technique can reduce statistical noise in the assessment of the correlation.

Referring back to FIGURE 4, in the further embodiment, the usage time series data and occupancy time series data are also evaluated to find a correlation between the usage of the indoor climate control system and the building's occupancy (Step 56), as further described below with reference to FIGURE 6. If the temperature correlation for a house indicates that there is little change in the thermostat set point over each day, assessment of whether that is an appropriate or efficient strategy of usage depends in part upon whether the occupancy varies over the day. If people are at home all day, leaving the indoor climate control running with the same thermostat set point all day may be appropriate. However, if all people leave the house during the day to go to work, changing the thermostat set point to reduce indoor climate control usage and hence energy consumption during the day may be both more appropriate and more efficient. Study of the correlation of occupancy and usage alongside the temperature correlation can give a remote indication of whether the thermostat set point behavior is reasonable and efficient based on the occupancy of the building. While the temperature correlation indicates whether the set point is being changed regularly, the occupancy correlation gives an indication of whether expecting that the set point be changed is appropriate.

Since human occupants are a latent and sensible heat load, with a fixed thermostat set point, air conditioning load is expected to increase with occupancy, which can be observed as a correlation between usage and occupancy. In contrast, with a fixed thermostat set point, heater load is expected to decrease with occupancy, which may be observed as a negative correlation between usage and temperature. To understand whether the thermostat is being adjusted with occupancy, such that, for example, the air conditioning thermostat set point is raised when occupancy is low and the air conditioning thermostat set point is lowered when occupancy is high, the temperature correlation must be compared to the correlation between usage and occupancy. A low correlation between temperature and usage, and a positive and high correlation between system usage and occupancy indicates that the thermostat set point is adjusted based on occupancy. In addition to the usage time series data and the temperature time series data, the occupancy time series data can be interpolated to a different time base. Similarly, data can be included (or excluded) by selecting operating cycles in the usage time series for only certain hours days, weeks, months, times of the year, or time periods, along with the corresponding temperatures in the temperature time series and the corresponding occupancies in the occupancy time series. Further correlations of system usage to other factors are possible, as well as correlations between ambient temperature and occupancy.

Occupancy of the building can be tracked in many different ways. Many commercial buildings already track occupancy from entry badge, electronic key, a computer network authorizations within the building. Occupancy, or changes in the occupancy, can also be directly sensed by:
- providing an entry sensor in an entryway for sensing ingress and egress of occupants of the building;
- providing a motion sensor within the building for sensing movement of the occupants of the building;
- providing an infrared sensor for sensing heat radiated from the occupants of the building;
- providing a humidity sensor in the building for sensing humidity generated by the occupants of the building;
- providing a noise level sensor in the building for detecting noise created by the occupants of the building;
- providing a carbon dioxide sensor in the building for detecting carbon dioxide created by the occupants of the building; and
- providing a vibration sensor in the building for detecting vibrations generated by the occupants of the building.

Still other types of sensors are possible. Further, occupancy, or changes in the occupancy, can be indirectly tracked by observing a frequency with which appliances in the building are operated, tracking energy usage in the building, and tracking wireless network usage in the building. Still other indirect indications of the occupancy, or changes in occupancy, are possible. Finally, demographic or work habit data can simply be obtained to represent the occupancy of the building. The demographic data can be used, for instance, to only count occupants of a certain age in the occupancy of the building or to make assumptions about occupancy of a house during workdays. Occupancy may include human occupants and animals, such as pets, or occupancy may be defined to only include human occupants.

Occupancy, when correlated with air conditioner usage and compared to temperature correlation, can indicate when manual adjustment of the thermostat set point is due to occupancy. FIGURE 6 is a flow diagram showing a routine for correlating indoor climate control system usage to occupancy 70 for use in the method 50 of FIGURE 4. As with the correlation between usage and temperature, the correlation between usage and occupancy can be established through the same aforementioned statistical analyses (step 71). Likewise, the correlation could represent a coefficient of correlation between a time series that includes a time 30 delay relative to the other time series, such as a delay between a change in occupancy and a change in the indoor climate control system usage, as occurs when people leave a building after turning down the thermostat set point. Finally, the correlation could be expressed as a coefficient of correlation between a finite difference or a derivative taken of the data in one of the time series and the finite difference or a derivative taken of the data in the other time series. Still other types of correlation are possible.

Correlating usage and occupancy follows a similar approach as the correlating usage and temperature. For instance, correlation between usage and occupancy can be established by characterizing the data in the usage time series and the temperature time series, as well as characterizing the data in the usage time series and the occupancy time series (step 72). The function that defines occupancy correlation can be, for instance, a statistical correlation of characteristic usage and characteristic temperature. In a further embodiment, the characteristic usages, characteristic temperatures, and characteristic occupancies can be screened using thresholds (step 73) that define a minimum amount or degree of change. In a still further embodiment, the characteristic usages and characteristic temperatures can be evaluated for regularly-occurring changes (step 74).

Finally, the relative strengths of the correlation between usage and temperature and the correlation between usage and occupancy can be compared. A positive and high correlation between usage and occupancy and a low correlation between usage and temperature strongly suggests that the thermostat set point is changed based on changing occupancy of the building. In addition, the frequency of the changing of the thermostat set point can be found, along with the frequency with which the occupancy of the building changes. The thermostat set point changing frequency can then be compared to the occupancy changing frequency, as a sanity check on the two correlations.

Referring back to FIGURE 4, the changing of the thermostat set point can be evaluated (Step 56). One type of evaluation is with respect to providing energy consumption reduction incentives. In particular, the finding of a pattern of changing of a building's thermostat set point can be used to distinguish among a group of utility customers to determine which ones should be targeted for certain interactions. For example, a customer that uses a lot of energy for air conditioning and who seemingly never changes his thermostat set point, that is, his assessed thermostat set point changing is low, compared to other customers, might first be offered an incentive for changing the thermostat set point whenever he leaves the house before being offered a weatherization package.

Another type of evaluation is with respect to providing energy consumption reduction incentives. Thus, an appropriate type of incentive can be chosen with respect to incenting a customer to regularly adjust their thermostat set point based on occupancy. For instance, an energy consumption reduction offering can be targeted to occupants of a building when the assessed thermostat set point changing fails to occur regularly in light of the changing of the occupancy of the building. As before, the effectiveness of the change in behavior, and therefore the effectiveness of the incentive, can be weighed by re-determining the thermostat set point adjustment patterns, and comparing "before" and "after" adjustment patterns.

In a broader sense, the findings of thermostat set point adjustment patterns can be used to distinguish among a group of utility customers to compare the adjustment patterns, and, if appropriate, determine which ones should be targeted for certain interactions. If a power utility is offering rebates, incentives, or education to the owner, occupant, or responsible party of a house related to adjusting indoor climate control systems, those opportunities might be offered first to those customers who, based on temperature and occupancy correlations to usage, are judged to be most infrequently changing their thermostat set points based on occupancy.

For example, energy usage data for the indoor climate control systems of the group could be obtained and compared. A customer within the group that uses a lot of energy for air conditioning can be evaluated to assess whether the customer adjusts his thermostat set point regularly and, if so, based on occupancy. Comparisons can be made based on indirect information, and based on these comparisons, a power utility is better able to target rebate and incentive programs. A power utility can also monitor the impacts of energy efficiency programs. The comparison can be used, for instance, to better understand why one or more of the buildings use more energy for indoor climate control than other buildings and to help narrow the set of likely reasons for excessive energy consumption.

The power utility can also use the comparison of thermostat set point adjustment patterns to choose which recommendations to make to building owners, occupants, or managers for reducing energy consumption. In addition, the findings can be used by third parties who want to advertise or offer products and services for reducing energy consumption or optimizing or changing indoor climate control to building owners, occupants, managers, or responsible parties. For example, a company that makes thermostats that are designed to encourage responsible setting of the thermostat set point when a building is occupied and resetting of the thermostat set point when the building is empty might want to target those customers with the worst thermostat set point adjustment patterns. Other uses of the comparison are possible.

The foregoing methodology, as described *supra* with reference to FIGURES 4-6, can be performed by one or more computers operating independently or over a network. FIGURE 7 is a block diagram showing a computer-implemented system 120 for externally evaluating thermostat adjustment patterns of an indoor climate control system in a building in accordance with one embodiment. The methodology can be implemented as a computer program 122 for execution by a personal computer 121 or similar computational device, which include components conventionally found in general purpose programmable computing devices, such as a central processing unit, memory, input/output ports, network interfaces, and non-volatile storage, although other components are possible.

The personal computer 121 can either operate stand-alone or be interconnected over a network 123, which could be a local area network, enterprise network, or wide area network, including the Internet, or some combination thereof. The personal computer 121 can include a storage device (not shown) or storage 124 can be provided over the network 123. The storage is used to store the energy usage data 125. In addition, a power utility (not shown) may maintain a storage device 128 to track their customers 129 and incentives 130. Other data can also be stored.

While the invention has been particularly shown and described as referenced to the embodiments thereof, those skilled in the art will understand that the foregoing and other changes in form and detail may be made therein without departing from the spirit and scope of the invention.

## Claims

1. A computer-implemented method for externally evaluating thermostat adjustment patterns of an indoor climate control system in a building based on temperature, comprising the steps of:
obtaining a usage time series that reflects indoor climate control system usage in a building over a plurality of operating cycles, each operating cycle comprising a "go-to-idle" state transition triggered by a thermostat during which the indoor climate control system transitions from a running state to an at idle state and a "go-to-run" state transition triggered by the thermostat during which the indoor climate control system transitions from an at idle state to a running state, the indoor climate control system running for a period of running time between each "go-to-run" state transition and the next "go-to-idle" state transition, the indoor climate control system remaining at idle for a period of idle time between each "go-to-idle" state transition and the next "go-to-run" state transition, the running time comprising the time necessary to bring the building's interior temperature into a temperature range defined about a desired indoor temperature as specified by a set point of the thermostat for the building;
obtaining a temperature time series for the temperature ambient to the building over the same plurality of the operating cycles; and
finding a temperature correlation between the usage time series and the temperature time series, wherein a low temperature correlation is interpreted as changing of the thermostat set point during the operating cycles,
wherein the steps are performed on a suitably-programmed computer.

2. A computer-implemented method for externally evaluating thermostat adjustment patterns of an indoor climate control system in a building based on temperature and occupancy, comprising the steps of:
obtaining a usage time series that reflects indoor climate control system usage in a building over a plurality of operating cycles, each operating cycle comprising a "go-to-idle" state transition triggered by a thermostat during which the indoor climate control system transitions from a running state to an at idle state and a "go-to-run" state transition triggered by the thermostat during which the indoor climate control system transitions from an at idle state to a running state, the indoor climate control system running for a period of running time between each "go-to-run" state transition and the next "go-to-idle" state transition, the indoor climate control system remaining at idle for a period of idle time between each "go-to-idle" state transition and the next "go-to-run" state transition, the running time comprising the time necessary to bring the building's interior temperature into a temperature range defined about a desired indoor temperature as specified by a set point of the thermostat for the building;
obtaining a temperature time series for the temperature ambient to the building over the same plurality of the operating cycles;
obtaining an occupancy time series for occupancy of the building over the same plurality of the operating cycles;
finding a temperature correlation between the usage time series and the temperature time series, wherein a low temperature correlation is interpreted as changing of the thermostat set point during the operating cycles; and
finding an occupancy correlation between the usage time series and the occupancy time series, wherein a high occupancy correlation coupled with the low temperature correlation is interpreted as the thermostat set point changing with changing of the occupancy of the building,
wherein the steps are performed on a suitably-programmed computer.

3. A method according to Claim 2, further comprising the steps of:
selecting a big time unit that is larger than the time unit used in the usage time series;
dividing the usage time series into increments of the big time unit;
forming groups of the usage data in the usage time series within each increment of the big time unit and finding characteristic usages that are representative of the usage data in each of the usage data groups;
forming groups of the temperature data in the temperature time series within each increment of the big time unit and finding characteristic occupancies that are representative of the temperature data in each of the temperature data groups;
forming groups of the occupancy data in the occupancy time series within each increment of the big time unit and finding characteristic occupancies that are representative of the occupancy data in each of the occupancy data groups;
defining the temperature correlation as a function of the characteristic usage of each of the usage data groups and the characteristic temperature of each of the temperature data groups; and
defining the occupancy correlation as a function of the characteristic usage of each of the usage data groups and the characteristic occupancy of each of the occupancy data groups.

4. A method according to Claim 3, further comprising of the steps of:
defining a threshold of usage change;
defining a threshold of temperature change;
defining a threshold of occupancy change;
identifying each of the times that the characteristic usage of each of the usage data groups comprised in consecutive increments of the big time unit exceed the usage change threshold as a series of usage changes;
identifying each of the times that the characteristic temperature of each of the temperature data groups comprised in consecutive increments of the big time unit exceed the temperature change threshold as a series of temperature changes;
identifying each of the times that the characteristic occupancy of each of the occupancy data groups comprised in consecutive increments of the big time unit exceed the occupancy change threshold as a series of occupancy changes;
comparing the usage changes series to the temperature changes series as the temperature correlation; and
comparing the usage changes series to the occupancy changes series as the occupancy correlation.

5. A method according to Claim 2, further comprising the step of:
retaining operating cycles in the usage time series for only select hours days, weeks, months, times of the year, or time periods, and also retaining only the temperatures in the temperature time series and only the occupancies in the occupancy time series that both correspond to the times of the retained operating cycles in the usage time series, prior to finding the temperature correlation and the occupancy correlation.

6. A method according to Claim 2, wherein the temperature correlation comprises at least one of a Pearson product-moment correlation coefficient, a rank correlation coefficient, a multi-moment correlation coefficient, a Brownian or distance correlation coefficient, a coefficient of correlation between two time series in which one of the time series comprises a time delay relative to the other time series, and a coefficient of correlation between a finite difference or derivative of one of the time series and a finite difference or derivative of the other time series, and the occupancy correlation comprises at least one of a Pearson product-moment correlation coefficient, a rank correlation coefficient, a multi-moment correlation coefficient, a Brownian or distance correlation coefficient, a coefficient of correlation between two time series in which one of the time series comprises a time delay relative to the other time series, and a coefficient of correlation between a finite difference or derivative of one of the time series and a finite difference or derivative of the other time series.

7. A method according to Claim 2, further comprising the steps of:
finding a thermostat set point frequency of the changing of the thermostat set point;
finding an occupancy frequency of changing of occupancy of the building; and
correlating the thermostat set point frequency to the occupancy frequency.

8. A method according to Claim 2, further comprising the step of:
choosing an energy consumption reduction offering with respect to the changing of the thermostat set point of the building.

9. A method according to Claim 8, further comprising the step of:
targeting the energy consumption reduction offering to occupants of the building when the assessed thermostat set point changing fails to occur regularly with the changing of the occupancy of the building.

10. A method according to Claim 2, further comprising determining the occupancy of the building by performing at least one of the steps of:
including only occupants of the building who are awake or exhibiting a level of activity as part of the occupancy of the building;
sensing occupancy or changes in the occupancy of the building, comprising at least one of the steps of:
providing an entry sensor in an entryway configured to sense ingress and egress of occupants of the building;
providing a motion sensor within the building configured to sense movement of the occupants of the building;
providing an infrared sensor configured to sense heat radiated from the occupants of the building;
providing a humidity sensor in the building configured to sense humidity generated by the occupants of the building;
providing a noise level sensor in the building configured to detect noise created by the occupants of the building;
providing a carbon dioxide sensor in the building configured to detect carbon dioxide created by the occupants of the building; and
providing a vibration sensor in the building configured to detect vibrations generated by the occupants of the building;
tracking a frequency with which appliances in the building are operated as an indication of the occupancy;
tracking energy usage in the building as an indication of the occupancy;
tracking wireless network usage in the building as an indication of the occupancy;
obtaining demographic data representing the occupancy of the building; and
determining occupancy from entry badge, electronic key, or computer network authorizations within the building.
